# EUROPEAN PATENT SPECIFICATION

(11) **EP 1 641 743 B1**
(45) Date of publication and mention of the grant of the patent: **12.11.2008**
(21) Application number: 04748775.6
(22) Date of filing: 01.07.2004
(51) Int. Cl.: C07C 231/08

(54) **PROCESS FOR IOHEXOL MANUFACTURE**
VERFAHREN ZUR HERSTELLUNG VON IOHEXOL
PROCEDE DE PRODUCTION D'IOHEXOL

(30) Priority: 03.07.2003 NO 20033058
(43) Date of publication of application: 05.04.2006
(73) Proprietor: GE Healthcare AS, 0401 Oslo (NO)
(72) Inventor: HOLMAAS, Lars, Terje, N-4510 Spangereid (NO); HOMESTAD, Ole, Magne, N-4510 Spangereid (NO); CERVENKA, Jan, GE Healthcare AS,, N-0401 Oslo (NO); HUSSAIN, Khalid, GE Healthcare AS,, N-0401 Oslo (NO)
(74) Representative: Flechsler, Insa
(86) International application number: PCT/NO2004/000199
(87) International publication number: WO 2005/003080

(56) References cited:
- WO-A-98/08804
- WO-A-99/26916
- US-A- 5 847 212
- HAAVALDSEN J ET AL: "X-RAY CONTRAST AGENTS. I. SYNTHESIS OF SOME DERIVATIVES OF 5-AMINO-2, 4, 6-TRIIODOISOPHTHLAMIDE" ACTA PHARMACEUTICA SUECICA, XX, XX, vol. 20, no. 3, 1983, pages 219-232, XP002052827 ISSN: 0001-6675

## Description

The present invention relates to a process for the manufacture of iohexol, 5-[N- (2,3-dihydroxypropyl) -acetamido]-N,N'-bis(2,3 -dihydroxypropyl)-2,4,6-triiodoisophtalamide.

lohexol is the non-proprietory name of the chemical drug substance of a non-ionic iodinated X-ray contrast agent marketed under the trade name OMNIPAQUE^{®}. OMNIPAQUE^{®} is one of the most used agents in diagnostic X-ray procedures.

The manufacture of such non-ionic contrast agents involves the production of the chemical drug substance (referred to as primary production) followed by formulation into the drug product (referred to as secondary production). Primary production of iohexol involves a multistep chemical synthesis and a thorough purification stage. For a commercial drug product it is important for the primary production to be efficient and economical and to provide a drug substance fulfillling the specifications.

The final step in the synthesis of iohexol is a N-alkylation step in which 5-(acetamido)-N,N'-bis(2,3-dihydroxypropyl)-2,4,6 triiodoisophtalamide (hereinafter 5-Acetamide) is reacted in the liquid phase with an alkylating agent to introduce the 2,3-dihydroxypropyl group at the nitrogen of the 5-acetamido group. Following this reaction, iohexol is isolated from the reaction mixture and purified by crystallisation and treatment with ion exchange resins.

The manufacture of iohexol is disclosed for example in US-4,250,113. In the last step of the multistep chemical synthesis crude iohexol is obtained from the reaction between 5-Acetamide and 1-chloro-2,3-propandiol at ambient temperature in propylene glycol and in the presence of sodium methoxide. The solvent is then evaporated and crude iohexol is obtained. The crude product is evaporated to dryness and recrystallised twice from butanol.

Several suggestions to improve the N-alkylation and the purification steps have been published. WO-A-98/08804 discloses the use of 2-methoxy-ethanol and optionally isopropanol both in the alkylation step of 5-Acetamide and in the purification of crude iohexol. WO-A-02/083623 discloses the purification of crude iohexol using 1-methoxy-2-propanol as the solvent optionally in a mixture with other solvents.

The N-alkylation step where 5-Acetamide in solution is reacted with an alkylation agent such as e.g. 1-chloro-2,3-propandiol to introduce the 2,3-dihydroxypropyl group at the nitrogen of the 5-acetamido group is illustrated in Scheme 1:

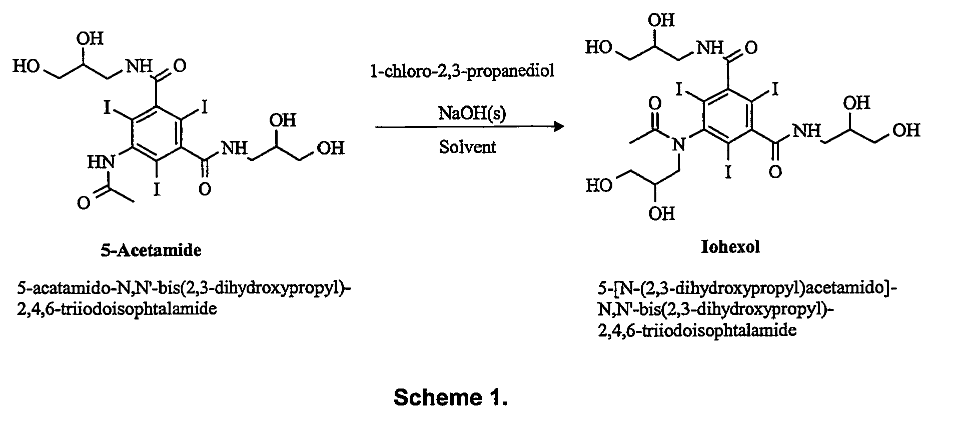

The N-alkylation step is challenging because O-alkylated by-products can also be formed when the alkylation occurs at the oxygen atoms of the hydroxy groups. It is therefore a desire to limit the formation of these O-alkylated by-products and thereby to limit their presence in the final purified iohexol. The upper limit for values for O-alkylated by-products in the end product is fixed by the European Pharmacopea to 0.6% (HPLC by area).

The O-alkylated by-products are removed to the degree desired or necessary by recrystallisation steps. Further unidentified by-products also referred to as impurities are also formed during the alkylation reaction and must be reduced to a tolerable level. In addition the solvents used should be easily available, be environmentally friendly and be of low toxicity.

There is therefore a need to identify a solvent that can be used in the N-alkylation reaction and that fulfil the desiderata mentioned above. It is further desired to improve the overall process including the N-alkylation step and the purification step in the manufacture of iohexol. If the crude product obtained by the N-alkylation step is to be re-crystallised from a solvent that is different from the solvent used in the N-alkylation step, then the reaction solvent must first be removed e.g. by evaporation to dryness. It is known from crystallisation theory and experience that even small quantities of residual solvents from previous steps may cause a crystallisation process to get out of control due to changes in its supersaturation conditions, and thorough removal of the reaction solvent is an important step. Solvent removal is an energy consuming operation which also risks degradation of the product due to exposure to elevated temperature.

The present invention improves the manufacture of iohexol meeting the needs discussed above.

It has now surprisingly been found that the use of a solvent comprising a C₁-C₅-monoalkylether of a C₃-C₁₀ alkylene-glycol in the N-alkylation of 5-Acetamide will give crude iohexol in almost quantitative yield.

It has further been found that by using a solvent comprising a C₁-C₅-monoalkylether of a C₃-C₁₀ alkylene-glycol both in the alkylation of 5-Acetamide and for the subsequent crystallisation of the resulting iohexol further improves the economy of the process and the overall yield and purity of the resulting iohexol product.

The specific characteristics of the invention will be evident from the patent claims.

In a first embodiment the invention provides a process for the production of iohexol comprising reacting 5-Acetamide with a 2,3-dihydroxypropylating agent in the presence of a base and of a solvent, the solvent comprising the C₁-C₅-monoalkylether of a C₃-C₁₀ alkylene-glycol. This process is illustrated in scheme 1 where the solvent comprises the C₁-C₅-monoalkylether of a C₃-C₁₀ alkylene-glycol.

In a preferred aspect of the invention the C₁-C₅₋monoalkylether of a C₃-C₁₀ alkylene-glycol is mixed with other solvents thus forming a solvent mixture. Such co-solvents are C₁-C₄ alkanols or water or mixtures of the alkanols and water. The co-solvents when used may constitute from 10 volume% to 60 volume% of the solvent mixture. A solvent consisting of the C₁-C₅-monoalkylether of a C₃-C₁₀ alkylene-glycol and a C₁-C₄ alkanol is preferred, and it is particularly preferred to use a solvent mixture where the C₁-C₄ alkanol is present in an amount of about 30 volume%. When water is used together with the C₁-C₅-monoalkylether of a C₃₋C₁₀ alkylene-glycol the water content of the solvent mixture should be up to 20 volume% and preferably about 10 volume%.

The preferred C₁-C₅-monoalkylether of a C₃-C₁₀ alkylene-glycol is 1-methoxy-2-propanol. Preferably 40-90 volume% of 1-methoxy-2-propanol is mixed with other solvents as outlined above. In a specifically preferred aspect a solvent mixture of 1-methoxy-2-propanol and up to 40 volume% methanol is used, with about 30 volume% methanol being most preferred. Alternatively a solvent mixture of 1-methoxy-2-propanol and up to 20 volume% water is used, with about 10 volume% of water being most preferred.

The solvents used in the process according to the invention are preferably used in an amount of 0.5-5ml per gram of 5-Acetamide, more preferred 0.7 to 3 ml per gram of 5-Acetamide and with 0.9-1.0 ml per gram of 5-Acetamide being the most preferred concentration.

The 2,3-dihydroxypropylating agent used in the N-alkylation reaction is any 2,3-dihydroxypropanol having a leaving group attached in the 1-position. 1-halo-2,3-propanediols are preferred with 1-chloro-2,3-propanediol being the most preferred 2,3-dihydroxypropylating agent. Alternatively, glycidol may be used as the 2,3-dihydroxypropylation agent. A molar excess of 2,3-dihydroxypropylation agent is preferably used e.g. 1.2-1.4 moles per mole of 5-Acetamide.

The N-alkylation step takes place in the presence of a base. The base used in the N-alkylation process according to the invention must be soluble in the reaction solvent. Alkali metal hydroxide is the preferred base and sodium hydroxide being most preferred.

The N-alkylation step is preferably effected at a temperature between 15-50°C, with 23-25°C being the most preferred process temperature.

The N-alkylation step will be allowed to proceed for several hours with a preferred reaction time of 12 to 48 hours and particularly preferred from 18 to 30 hours. The reaction may be terminated by quenching with an acid. Inorganic or organic acids may be used and inorganic acids such as HCl are preferred. The reaction may be monitored, e.g. by HPLC, to determine the appropriate stage at which quenching should take place.

Following termination of the reaction, the crude iohexol reaction product may be separated from the solvent e.g. by cooling and/or solvent evaporation. The evaporation should preferably be performed under reduced pressure. Usually most of the solvent from the N-alkylation step is distilled off, to produce a concentrated solution from which iohexol is further separated. The separation of the solvent is usually performed after the reduction of the salt content of the reaction mixture is performed.

After quenching the salt produced in the N-alkylation step is optionally reduced before the isolation of iohexol or further purification is started. The optionally reduction of the salt content is done without the need of removal of the solvents from the N-alkylation step. The bulk amount of salt can be removed by precipitation and filtering followed, if necessary, by treatment with a cationic ion exchange resin and an anionic ion exchange resin. The resulting iohexol in solution may be isolated by crystallisation directly from the solvents by evaporation of the solvents preferably by distillation under reduced pressure or iohexol may be further purified. By using the solvent or solvent mixture of the invention it is possible to obtain iohexol with a purity that satisfies the criteria of the European Pharmacopea of 0.6% (or less) of O-alkylated by-products without further recrystallisation procedures. In a final step iohexol is usually washed with isopropanol.

Although iohexol may be obtained directly from the N-alkylation reaction in a purity sufficient to satisfy the criteria of the European Pharmacopeia as outlined above, it is frequently desirable or necessary to further purify the product from the N-alkylation. Hence, in a further aspect of the invention the iohexol from the N-alkylation step optionally where the salt content is reduced (denoted "crude iohexol") is further purified using a solvent comprising a C₁-C₅-monoalkylether of a C₃-C₁₀ alkylene-glycol. In a preferred aspect the same C₁-C₅-monoalkylether of a C₃-C₁₀ alkylene-glycol as is used in the N-alkylation step is also used in the purification step and optionally together with a C₁-C₄ alkanol as co-solvent.

The solvent amounts are adjusted to 1.5-8 ml of the C₁-C₅-monoalkylether of a C₃-C₁₀ alkylene-glycol/g iohexol and 0-1 ml of the C₁-C₄ alkanol/g iohexol and the water content is reduced to 0.001-0.3 ml water/g iohexol. 1-methoxy-2-propanol is preferably used, however a solvent mixture such as a mixture of 1-methoxy-2-propanol with methanol may also be used in the concentrations of 1.5-8 ml preferably 2-6 ml of 1-methoxy-2-propanol/g iohexol, 0-1 ml methanol/g iohexol and a water content of 0.001-0.3 ml water/g iohexol.

The purification is preferably performed by the crystallisation of iohexol from the solvent or solvent mixture and separation of the crystals from the solvent. One crystallisation is usually sufficient to provide iohexol of a very good quality exceeding the criteria of the European Pharmacopeia. Starting with the solution of crude iohexol from the N-alkylation process and where excess salt has been removed where necessary by precipitation and/or ion exchange procedures, excess water is removed preferably by azeotropic distillation. The solvent amounts are adjusted during the distillation to satisfy the limits given above and the solution is seeded with iohexol crystals. The seeded solution is stirred with reflux and the volume is adjusted by distillation under reduced pressure. The solution is cooled under stirring and the crystalline iohexol is filtered off. The crystalline product is preferably washed with isopropanol and dried.

The purification process is preferably effected at a temperature range 50-130°C with a temperature range of 60-120° being most preferred. The purification of iohexol according to the invention is preferably effected over a time of approximately 4 hours to 2 days.

An important aspect of the improved quality obtained with the process according to the present invention and in particular when the N-alkylation step is followed by a further purification step is the ability to reduce the amount of O-alkylated by-products. The O-alkylated compounds are impurities that are difficult to remove in the purification process. The European Pharmacopea iohexol specification of an upper limit of 0.6% (by HPLC area) is one of the limiting factors in the process for obtaining a final product of necessary quality. It is therefore of critical importance to avoid formation of such O-alkylated by-products as much as possible in the N-alkylation process. Further, the enhanced purification effect by using the specific solvents of the invention makes it possible to obtaining an average of approximately 0.45% O-alkylated by-products instead of 0.51 to 0.55% O-alkylated by-products as was obtained in WO 98/08804 from an average crude iohexol process stream. This reduction is of great importance. The ability to obtain a final iohexol product with an amount of O-alkylated by-products well beyond the limits of the European Pharmacopea provide the producer with the ability to optimise other important process parameters and thereby increase e.g. the yield and to accept some batch variation and still obtain a product that satisfies the criteria for iohexol of the European Pharmacopea.

The invention will hereinafter be illustrated by the non-limiting examples. All percent are in HPLC area % when not stated otherwise.

### Example 1: Synthesis of iohexol in 1-methoxy-2-propanol/methanol

1-methoxy-2-propanol (44 ml), methanol (19 ml) and sodium hydroxide (4.87 g) was added to a jacketed glass reactor and stirred for about 15 minutes at 25°C. 5-Acetamide (70 g) was added to the reactor, and the mixture stirred overnight at 45°C, before it was allowed to cool to 25°C. 1-chloro-2,3-propanediol (12.43 g) was added to the solution. After 1.5 hours, more 1-chloro-2,3-propanediol (0.83 g) was added, and the reaction was allowed to proceed for 24 hours. HPLC analysis (water/acetonitrile) of the reaction mixture gave the following results:

| | |
|---|---|
| lohexol | 98.1 % |
| 5-Acetamide | 1.17 % |
| O-alkylated substances | 0.58 % |
| Other impurities | 0.1 % |

### Example 2: Synthesis of iohexol in 1-methoy-2-propanol/water

1-methoxy-2-propanol (63 ml), water (7 ml) and sodium hydroxide (4.50 g) was added to a jacketed glass reactor and stirred for about 15 minutes at 25°C. 5-Acetamide (70 g) was added to the reactor, and the mixture stirred overnight at 45°C, before it was allowed to cool to 35°C. 1-chloro-2,3-propanediol (11.39 g) was added to the solution. After 3 hours, more 1-chloro-2,3-propanediol (0.83 g) was added, and the reaction was allowed to proceed for 24 hours. HPLC analysis (water/acetonitrile) of the reaction mixture gave the following results:

| | |
|---|---|
| lohexol | 98.3 % |
| 5-Acetamide | 0.68 % |
| O-alkylated substances | 0.81 % |
| Other impurities | 0.3 % |

### Example 3: Alkylation and crystallisation in solutions containing 1-methoxy-2-propanol

1-methoxy-2-propanol (63 L), methanol (27 L) and sodium hydroxide (6.96 kg) was added to a 500 L reactor and stirred until all solids were dissolved and the temperature was below 30°C. 5-Acetamide (100 kg) was added to the reactor, and the mixture stirred overnight at 45°C before it was allowed to cool to 25°C. 1-chloro-2,3-propanediol (16.76 kg) was added to the clear solution. After 1.5 hours, more 1-chloro-2,3-propanediol (1.18 kg) was added, and the reaction was allowed to proceed for 30 hours. HPLC analysis (water/acetonitrile) of the reaction mixture gave the following results:

| | |
|---|---|
| lohexol | 97.9 % |
| 5-Acetamide | 0.9 % |
| O-alkylated substances | 0.83 % |
| Other impurities | 0.4 % |

The reaction was stopped by addition of hydrochloric acid (650 ml), and the reaction mixture diluted with a mixture of 1-methoxy-2-propanol (53 L) and methanol (13 L). The mixture was filtered, and the salts on the filter washed with methanol (3x10 L). The combined filtrate and wash was diluted with water (22 L) and treated with cationic ion exchange resin (AMB 200C, 80 L) and anionic ion exchange resin (IRA 67, 80 L) to a salt content of 0.006 w/w %. The solution was filtered, and the ion exchange resins washed in several stages with a mixture of water (160 L) and methanol (85 L). The combined filtrate and wash was concentrated under reduced pressure to a volume of 155 L. One half of this was taken further to crystallisation as described below.

Water was removed from the solution by azeotropic distillation. The volume was held at a constant level by replacing the distillate by 1-methoxy-2-propanol (80 L). At water content of 0.16 L/kg iohexol, further 1-methoxy-2-propanol (159 L) was added, and the solution seeded with iohexol crystals (0.26 kg). After stirring at reflux overnight, the volume of the solution was reduced by 42 L by distillation under reduced pressure (300-600 mbar). The temperature was set to 90°C, which was held for 3 hours before cooling to 60°C over 3 hours. The crystallisation mixture was stirred overnight at 60°C, filtered and washed with isopropanol (90 L, 6 portions). The yield was 48.4 kg (as dry powder), corresponding to 88-weight % corrected for seeding material and samples. HPLC analysis (water/acetonitrile) of the crystals gave the following results:

| | |
|---|---|
| lohexol | 99.3 % |
| 5-Acetamide | 0.15 % |
| O-alkylated substances | 0.45 % |
| Other impurities | 0.11 % |

## Claims

1. A process for the production of iohexol comprising alkylating 5-(acetamido)-N,N'-bis(2,3-dihydroxypropyl)-2,4,6-triiodoisophtalamide with a 2,3-dihydroxypropylating agent in the presence of a base and of a solvent which solvent comprises a C₁-C₅-monoalkylether of a C₃-C₁₀ alkylene-glycol.

2. A process as claimed in claim 1 wherein said glycol is 1-methoxy-2-propanol.

3. A process as claimed in claim 1 or 2 further comprising one or more co-solvents.

4. A process as claimed in claim 3 wherein said co-solvents comprise C₁-C₄ alkanols, preferably methanol, and/or water.

5. A process as claimed in claim 3 or 4 wherein said solvent comprises 1-methoxy-2-propanol and 0-40 volume% of methanol.

6. A process as claimed in claim 3 or 4 wherein said solvent comprises 1-methoxy-2-propanol and 0-20 volume% of water.

7. A process as claimed in claims 1 to 6 wherein said solvent is used in an amount of 0.5 to 5 ml, more preferred 0.7 to 3 ml and most preferred 0.9 to 1.0 ml per gram 5-Acetamide.

8. A process as claimed in any of the previous claims further comprising purifying the crude iohexol obtained from the N-alkylation reaction using a solvent comprising a C₁-C₅-monoalkylether of a C₃-C₁₀ alkylene-glycol.

9. A process as claimed in claim 8 where the C₁-C₅-monoalkylether of a C₃-C₁₀ alkylene-glycol is the same glycol as used in the N-alkylation process.

10. A process as claimed in claims 8 and 9 wherein in said purification the C₁-C₅-monoalkylether of a C₃-C₁₀ alkylene-glycol is 1-methoxy-2-propanol.

11. A process as claimed in claims 8 to 10 wherein in said purification the solvent further comprises one or more co-solvents.

12. A process as claimed in claim 11 wherein said co-solvent comprises C₁-C₄ alkanols and preferably methanol.

13. A process as claimed in claims 9 to 12 wherein the amount of said solvent is adjusted to 1.5 to 8 ml of the C₁-C₅-monoalkylether of a C₃-C₁₀ alkylene-glycol/g iohexol, to 0-1 ml C₁-C₄ alkanol/g iohexol, and to 0.001-0.3 ml water/g iohexol.

14. A process as claimed in claims 8 to 13 where the purification is performed by crystallising the iohexol from said solvent and then separating the crystals from said solvent.

15. A process as claimed in claims 8 to 14 wherein the salt content in the reaction mixture of the alkylation reaction is reduced prior to the purification step.

16. A process as claimed in claims 8 to 15 wherein the water content in the reaction mixture of the alkylation reaction is reduced prior to the crystallisation step preferably by azeotropic distillation.

17. A method as claimed in claims 8 to 16 where the crystalline iohexol is washed with isopropanol and dried.

## Patentansprüche

1. Verfahren zur Herstellung von lohexol, umfassend das Alkylieren von 5-(Acetamido)-N,N'-bis(2,3-dihydroxypropyl)-2,4,6-triiodisophthalamid mit einem 2,3-Dihydroxypropylierungsmittel in Gegenwart einer Base und eines Lösungsmittels, welches Lösungsmittel einen C₁-C₅-Monoalkylether eines C₃-C₁₀-Alkylen-Glykols umfasst.

2. Verfahren nach Anspruch 1, wobei es sich bei dem Glykol um 1-Methoxy-2-propanol handelt.

3. Verfahren nach Anspruch 1 oder 2, welches weiterhin ein oder mehrere Co-Lösungsmittel umfasst.

4. Verfahren nach Anspruch 3, wobei die Co-Lösungsmittel C₁-C₄-Alkanole, vorzugsweise Methanol, und/oder Wasser umfassen.

5. Verfahren nach Anspruch 3 oder 4, wobei das Lösungsmittel 1-Methoxy-2-propanol und 0-40 Vol.-% Methanol umfasst.

6. Verfahren nach Anspruch 3 oder 4, wobei das Lösungsmittel 1-Methoxy-2-propanol und 0-20 Vol.-% Wasser umfasst.

7. Verfahren nach Anspruch 1 bis 6, wobei das Lösungsmittel in einer Menge von 0,5 bis 5 ml, besonders bevorzugt 0,7 bis 3 ml und ganz besonders bevorzugt 0,9 bis 1,0 ml pro Gramm 5-Acetamid verwendet wird.

8. Verfahren nach einem der vorstehenden Ansprüche, weiterhin umfassend das Reinigen des rohen lohexols, erhalten aus der N-Alkylierungsreaktion, unter Verwendung eines Lösungsmittels, das einen C₁-C₅-Monoalkylether eines C₃-C₁₀-Alkylen-Glykols umfasst.

9. Verfahren nach Anspruch 8, wobei der C₁-C₅-Monoalkylether eines C₃-C₁₀-Alkylen-Glykols das gleiche Glykol ist, wie im N-Alkylierungsprozess verwendet.

10. Verfahren nach Anspruch 8 und 9, wobei der C₁-C₅-Monoalkylether eines C₃-C₁₀-Alkylen-Glykols bei der Reinigung 1-Methoxy-2-propanol ist.

11. Verfahren nach Anspruch 8 bis 10, wobei bei der Reinigung das Lösungsmittel weiterhin ein oder mehrere Co-Lösungsmittel umfasst.

12. Verfahren nach Anspruch 11, wobei das Co-Lösungsmittel C₁-C₄-Alkanole und vorzugsweise Methanol umfasst.

13. Verfahren nach Anspruch 9 bis 12, wobei die Menge des Lösungsmittels reguliert wird auf 1,5 bis 8 ml des C₁-C₅-Monoalkylethers eines C₃-C₁₀-Alkylen-Glykols/g lohexol, auf 0-1 ml C₁-C₄-Alkanol/g lohexol und auf 0,001-0,3 ml Wasser/g lohexol.

14. Verfahren nach Anspruch 8 bis 13, wobei die Reinigung durch Kristallisieren des lohexols aus dem Lösungsmittel und dann durch Abtrennen der Kristalle vom Lösungsmittel durchgeführt wird.

15. Verfahren nach Anspruch 8 bis 14, wobei der Salzgehalt im Reaktionsgemisch der Alkylierungsreaktion vor dem Reinigungsschritt verringert wird.

16. Verfahren nach Anspruch 8 bis 15, wobei der Wassergehalt im Reaktionsgemisch der Alkylierungsreaktion vor dem Kristallisierungsschritt vorzugsweise durch azeotrope Destillation verringert wird.

17. Verfahren nach Anspruch 8 bis 16, wobei das kristalline lohexol mit Isopropanol gewaschen wird und getrocknet wird.

## Revendications

1. Procédé de production d'iohexol comprenant l'alkylation de 5-(acétamido)-N,N'-bis(2,3-dihydroxypropyl)-2,4,6-triiodoisophtalamide avec un agent de 2,3-dihydroxypropylation en la présence d'une base et d'un solvant, lequel solvant comprend un mono-alkyléther en C₁-C₅ d'un alkylène-glycol en C₃-C₁₀.

2. Procédé selon la revendication 1 dans lequel ledit glycol est du 1-méthoxy-2-propanol.

3. Procédé selon la revendication 1 ou 2 comprenant, en outre, un ou plusieurs solvants simultanés.

4. Procédé selon la revendication 3 dans lequel lesdits solvants simultanés comprennent des alcanols en C₁-C₄, de préférence, du méthanol, et/ou de l'eau.

5. Procédé selon la revendication 3 ou 4 dans lequel ledit solvant comprend du 1-méthaxy-2-propanol et de 0 à 40 % en volume de méthanol.

6. Procédé selon la revendication 3 ou 4 dans lequel ledit solvant comprend du 1-méthoxy-2-propanol et de 0 à 20 % en volume d'eau,

7. Procédé selon les revendications 1 à 6 dans lequel ledit solvant est utilisé en une quantité de 0,5 à 5 ml, de manière plus préférée, de 0,7 à 3 ml et de manière la plus préférée de 0,9 à 1,0 ml par gramme de 5-acétamide.

8. Procédé selon l'une quelconque des revendications précédentes comprenant, en outre, la purification de l'iohexol brut obtenu à partir de la réaction de N-alkylation en utilisant un solvant comprenant un mono-alkyléther en C₁-C₅ d'un alkylène-glycol en C₃-C₁₀.

9. Procédé selon la revendication 8 dans lequel le mono-alkyléther en C₁-C₅ d'un alkylène-glycol en C₃-C₁₀ est le même glycol qu'utilisé dans le traitement de N-alkylation.

10. Procédé selon les revendications 8 et 9 dans lequel, dans ladite purification, le mono-alkyléther en C₁-C₅ d'un alkylène-glycol en C₃-C₁₀ est le 1-méthoxy-2-propanol.

11. Procédé selon les revendications 8 à 10 dans lequel, dans ladite purification, le solvant comprend, en outre, un ou plusieurs solvants simultanés.

12. Procédé selon la revendication 11 dans lequel ledit co-solvant comprend des alcanols en C₁-C₄ et, de préférence, du méthanol.

13. Procédé selon les revendications 9 à 12 dans lequel la quantité dudit solvant est ajustée de 1,5 à 8 ml du mono-alkyléther en C₁-C₅ d'un alkylène-glycol en C₃-C₁₀ /g de iohexol, de 0 à 1 ml d'alcanol en C₁-C₄ /g de iohexol, et de 0,001 à 0,3 ml d'eau/g de iohexol.

14. Procédé selon les revendications 8 à 13 dans lequel la purification est réalisée en cristallisant l'iohexol provenant dudit solvant puis en séparant les cristaux dudit solvant.

15. Procédé selon les revendications 8 à 14 dans lequel la teneur en sel dans le mélange de réaction de la réaction d'alkylation est réduite avant l'étape de purification.

16. Procédé selon les revendications 8 à 15 dans lequel la teneur en eau dans le mélange de réaction de la réaction d'alkylation est réduite avant l'étape de cristallisation, de préférence, par distillation azéotropique.

17. Procédé selon les revendications 8 à 16 dans lequel l'iohexol cristallin est purifié avec de l'isopropanol et séché.
